# FASCICULE DE BREVET EUROPEEN

(11) **EP 0 327 410 B1**
(45) Date de publication et mention de la délivrance du brevet: **31.08.1994**
(21) Numéro de dépôt: 89400020.7
(22) Date de dépôt: 04.01.1989
(51) Int. Cl.: A61B 17/32, A61B 17/36

(54) **Dispositif de coelioscopie opératoire multifonctionnel permettant d'effectuer différents gestes opératoires avec introduction d'instruments**
Multifunktionelles Operationsinstrument für die Laparoskopie, das unter Einführung von Instrumenten verschiedene Operationsakte erlaubt
Multifunctional operatory laparoscopy device permitting to execute different operatory actions with instruments introduction

(30) Priorité: 05.01.1988 FR 8800170
(43) Date de publication de la demande: 09.08.1989
(73) Titulaire: Karl Storz GmbH & Co., D-78532 Tuttlingen (DE)
(72) Inventeur: Manhes, Hubert, F-03200 Vichy (FR); Ognier, Jean-François, Aulhac F-15240 Saignes (FR)
(74) Mandataire: Chanet, Jacques

(56) Documents cités:
- EP-A- 0 172 490
- DE-U- 8 225 493
- FR-A- 2 474 857
- US-A- 4 597 380

## Description

La présente invention est du domaine des appareils chirurgicaux et elle a pour objet un instrument multifunctionnel pour les interventions de chirurgie endoscopique, dite aussi coelioscopique.

On connaît par une publication FR 2.474.857 (Ets BOUTMY, Inv. MANHES) un appareil chirurgical plurifonctionnel comprenant un tube combinant un espace de circulation de fluide et de guidage d'instruments chirurgicaux, l'espace de circulation de fluide étant subdivisé en plusieurs sous-espaces donc au moins un conduit d'aspiration de liquide. Dans cet appareil un tube, dit canule, est inséré, au voisinage de l'une de ses extrémités, dans un boitier formant poignée de manipulation ; des ajutages latéraux débouchent dans la canule au niveau de la poignée ; toujours au niveau de la poignée, on peut introduire dans la canule un instrument tel que bistouri électrique. Cet appareil de l'art antérieur a fait les preuves de son efficacité ; cependant, son utilisation a permis de relever un certain nombre d'inconvénients ou d'insuffisances : limitation à trois du nombre des fonctions (incision, irrigation, aspiration), difficulté de stérilisation puisque c'est la totalité de l'appareil qui doit être portée à 200°C, nécessité de multiplier le nombre des incisions pour l'introduction d'autres instruments (ciseaux, pinces, laser, électrocoagulateurs, etc...).

On pourra également, pour connaître l'environnement de la technique proposée par la présente invention se reporter aux publications suivantes : US 3.902.495 décrivant un dispositif de contrôle et de régulation d'un système d'aspiration, US 3.920.014 décrivant un dispositif chirurgical permettant le contrôle de l'irrigation et de l'aspiration de fluides ; US 3.990.456 et US 4.030.502 décrivant des résectoscopes à électrodes bipolaires par voies urinaires ; enfin la publication US 4.614.625 décrivant une canule gastrique.

Le but général de la présente invention, à la lumière de l'expérience apportée par l'utilisation de l'appareil inventé par MANHES, et en raison de l'évolution des techniques connexes et des matériaux nouveaux apparus sur le marché, est de proposer un appareil chirurgical perfectionné visant à surmonter les inconvénients et insuffisances rencontrés avec l'appareil précité.

Un premier but particulier de l'invention est de mettre à la disposition du chirurgien un appareil bien ergonomique facilitant sa manipulation et améliorant la précision de son geste.

Un deuxième but particulier de l'invention est de proposer un appareil qui soit facilement nettoyable et stérilisable pour ses parties en contact direct ou indirect avec les tissus de l'organisme.

Un troisième but particulier est de proposer un appareil qui autorise l'adaptation et la mise en oeuvre rapides et précises d'une grande variété d'instruments chirurgicaux proprement dits (pinces, bistouri, etc...) ou d'instruments d'observation (coelioscope optique, micro-caméra, etc...).

Ainsi, et selon la présente invention, un appareil chirurgical destiné à la coelioscopie opératoire, dit instrument multifonctionnel, constitué d'un tube, à l'intérieur duquel peuvent être introduits différents instruments, ledit tube pouvant permettre le passage de liquides corporels ou d'irrigation et comportant au voisinage de son extrémité, dite externe, un ensemble, dit poignée, de manoeuvre et de raccordement;
- le tube, précité est constitué d'au moins deux parties : une première partie entourée par ladite poignée, une deuxième partie formant une canule, les deux parties pouvant être raccordées l'une à l'autre, dans leur prolongement axial, ou, alternativement, être séparées l'une de l'autre.
- ladite poignée supporte une pluralité de moyens de commandes des diverses fonctions, actionnables simultanément ou séparément par deux doigts de la même main, à savoir :
   . des premiers et deuxièmes moyens de commande électrique de l'envoi de liquide d'irrigation d'une part, et d'aspiration du liquide d'irrigation ou de liquides corporels d'autre part,
   . des troisièmes moyens de commande électrique, soit d'un coagulateur thermique ou électrique, soit d'un laser chirurgical,
   . des moyens, dits quatrièmes, de commande mécanique d'instruments rigides dits spécifiques, et/ou d'instruments dits souples.

Il résulte de cette disposition que la deuxième partie qui sera celle en contact direct ou indirect avec les tissus peut être nettoyée et stérilisée séparément de la première et il en résulte par voie de conséquence que la première partie peut être d'une certaine complexité dans ses composants mécaniques ou électriques.
L'appareil peut recevoir simultanément des instruments dits souples (pointe électrocoagulante monopolaire, lame coupante, fibre optique conductrice de faisceau Laser, etc...) et des instruments rigides qui peuvent être soit des instruments conventionnels (pinces, ciseaux, Laser CO₂, ponction étanche, etc...) comportant leurs propres moyens de manoeuvre, soit des instruments dits spécifiques, conçus pour être manoeuvrés depuis la poignée.

De manière avantageuse, le raccordement de la poignée à la canule est réalisé par le moyen d'un emboîtement à baïonnette "quart de tour" et rampe hélicoïdale, un mécanisme de verrouillage par cliquet assurant le maintien en position raccordée des deux parties de l'appareil.

De façon générale encore, la canule comporte au moins trois conduits le long de son axe : un conduit d'irrigation, un conduit de passage d'instruments de sectionnement souples de petit diamètre, un conduit axial de guidage d'instruments rigides, pouvant former aussi conduit d'évacuation ; en outre, la canule est solidaire d'une pièce dite de raccordement, approximativement en forme générale de prisme à base polygonale et comportant plusieurs faces latérales et deux faces de base, l'une des faces latérales, perpendiculaire à l'axe de la canule, constituant une face de raccordement à la poignée.

Avantageusement, le conduit axial bifurque dans la pièce de raccordement, d'une part en un conduit axial émergeant à la face de raccordement, d'autre part, en un conduit latéral d'évacuation émergeant en un premier ajutage sur une première face latérale, dite de branchement, de la pièce de raccordement ; avantageusement toujours, une vanne à tiroir pouvant alternativement obturer ou libérer le passage dans le conduit axial, est disposée en travers du conduit axial, l'axe du tiroir de la vanne étant perpendiculaire à l'axe du conduit axial et suffisamment proche de la bifurcation précédente pour ne pas créer de volume mort de rétention, tandis qu'un clapet situé dans un second ajutage, dit d'entrée d'irrigation, rend unidirectionnel en direction de la canule le passage de liquide tout en garantissant une pression minimale d'irrigation, pouvant donner lieu à un jet destiné à détacher et évacuer des particules organiques.

Avantageusement le conduit de guidage est une partie cylindrique du conduit axial et il comporte latéralement au moins une partie excentrée formant le conduit d'évacuation qui débouche dans le conduit latéral d'évacuation.

Avantageusement encore, le conduit de passage d'instruments souples débouche à ladite face de raccordement en coïncidence, lorsque les deux parties de l'appareil sont raccordées, avec son prolongement dans la poignée jusqu'à son débouché à une extrémité supérieure de cette dernière.

Suivant une autre disposition générale de l'invention, la poignée a la forme générale d'un parallélépipède allongé dont l'une des faces de base constitue une face de raccordement, et est perpendiculaire à ladite première partie du tube, l'axe de la poignée étant incliné d'environ 15° par rapport à l'axe du tube, et au moins un poussoir est disposé sur une face, dite postérieure, de la poignée tandis qu'un autre poussoir, dit détente, est disposé sur une face dite antérieure de la poignée, poussoirs et détente étant situés au voisinage de l'extrémité supérieure de cette dernière, tandis qu'une manette de manoeuvre est disposée sur l'une des faces latérales de la poignée au voisinage de l'extrémité supérieure de celle-ci.

Les poussoirs, dits plus haut premiers et deuxièmes moyens de commande, des préférence au nombre de deux disposés côte à côte, sont destinés l'un à la commande d'une électrovanne d'irrigation, l'autre à la commande d'une électrovanne d'aspiration ; les poussoirs sont accessibles au pouce, séparément ou simultanément.

La détente, dite plus haut troisièmes moyens de commande, est accessible à l'index ; elle est destinée à commander la mise sous tension ou le fonctionnement de divers appareils ou accessoires électriques.

La manette constituant les sus-nommés quatrième moyens de commande mécanique, est destinée à la manoeuvre manuelle d'un instrument rigide, dit spécifique, placé dans le conduit de guidage et/ou d'un instrument souple placé dans le conduit de passage ; la manette est reliée à un coulisseau entourant ladite première partie du tube dans un logement approprié de la poignée, en vue de communiquer, par l'intermédiaire du coulisseau et d'au moins un ergot intérieur de celui-ci passant à travers une lumière longitudinale pratiquée dans le tube, un mouvement axial à un organe d'un instrument rigide spécifique placé dans le tube.

Le coulisseau, percé lui-même axialement dans le prolongement dudit conduit de passage d'instruments souples, supporte un mécanisme d'accouplement destiné à lui accoupler un instrument souple passant dans le conduit de passage, et à permettre la manoeuvre en va-et-vient sous la dépendance de la manette, dudit instrument souple, ou, éventuellement, de découpler l'instrument souple du coulisseau en cas de présence dans le tube d'instruments rigides non compatibles avec l'instrument souple, le couplage ou le découplage étant opérés par un doigt palpeur faisant saillie à travers une lumière dans le tube.

La présente invention sera mieux comprise et des détails en relevant apparaîtront à la description qui va être faite d'une forme de réalisation en relation avec les figures des planches annexées dans lesquelles :
- les fig.1 et 2 sont des représentations en perspective sous deux angles différents d'un appareil chirurgical de l'invention,
- les fig.3 et 4 sont des coupes axiales respectivement des première et deuxième parties de l'appareil,
- les fig.5 et 6 sont des vues de côté correspondant respectivement aux fig.3 et 4 suivant les directions respectives A,B,
- la fig.7 est une coupe transversale, à plus grande échelle, suivant le plan CC de la fig.4,
- la fig.8 est une vue de dessus de la première partie suivant la direction D de la fig.5,
- la fig.9 est une vue de dessus suivant la direction E de la fig.6,
- la fig.10 est une coupe transversale par un plan FF de la fig.6,
- la fig.11 illustre, à échelle très agrandie, l'articulation de la manette visible sur les fig.3 et 5,
- la fig.12 est une coupe partielle suivant un plan GG de la figure précédente,
- la fig.13 est, à plus grande échelle que celle de la fig.3, une coupe par un plan transversal vue suivant la direction H de cette figure,
- la fig.14 est une coupe axiale correspondant à la fig.13 vue suivant la direction G de cette dernière figure,
- les fig.15 et 16 sont des coupes axiales à 90° l'une de l'autre de la partie du conduit principal continu dans la poignée,
- les fig.17 et 18 représentent de façon analogue la partie supérieure de deux instruments rigides spécifiques, les fig.15 à 18 permettant de comprendre le mode de couplage et de manoeuvre de l'instrument rigide, et
- la fig.19 est une représentation schématique de l'ensemble de l'installation et de divers accessoires utilisables avec l'instrument multifonctionnel de l'invention.

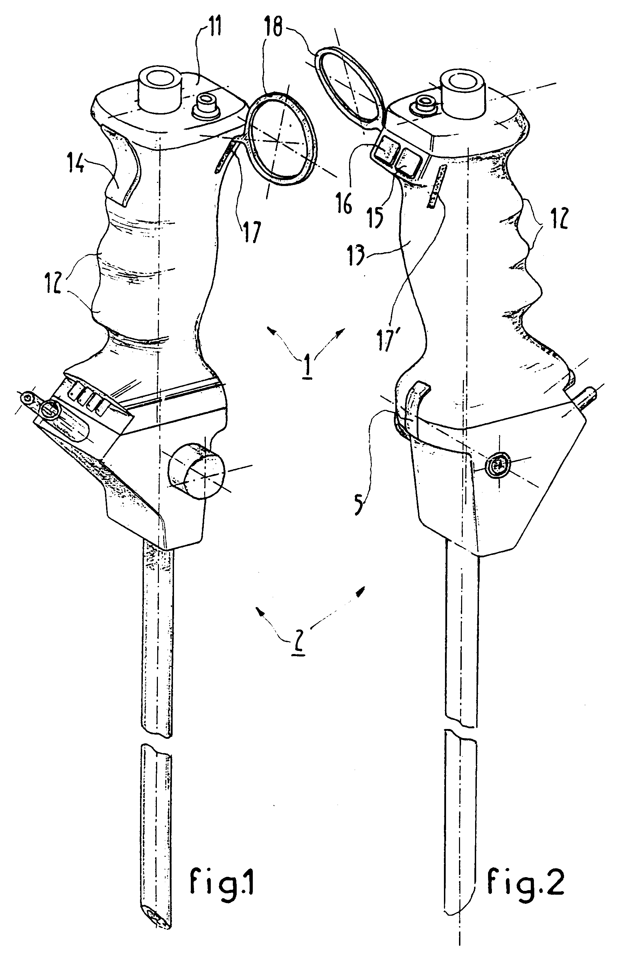

Sur les fig.1 à 6, la forme préférée de réalisation de l'appareil chirurgical de l'invention, apparaît composée de deux parties 1 et 2, dites respectivement poignée et canule pouvant être raccordées l'une à l'autre dans leur prolongement axial ; le raccordement s'effectue suivant deux faces de raccordement 3,3′ par le moyen d'un emboitement 4,4′ à baïonnette "quart de tour" et rampe hélicoïdale, un mécanisme de verrouillage par cliquet 5 assurant le maintien des parties 1 et 2 en position raccordée.

La première partie, supérieure,de l'appareil est constituée principalement par un premier tronçon 6 d'un tube de guidage entouré par la poignée proprement dite 8 ; la poignée proprement dite a une forme générale de parallélépipède allongé incliné d'environ une quinzaine de degrés par rapport à l'axe 9 du tube principal, les faces d'extrémité inférieure 3 et supérieure 11 étant perpendiculaires à l'axe 9 ; la poignée est un corps creux en deux parties moulées supportant ou contenant divers organes de commande ; on remarquera la forme ergonomique de la poignée, en particulier les sculptures 12 de prise des doigts de la face antérieure et la forme lisse et galbée de la partie postérieure 13, assurant un positionnement précis de la poignée par rapport aux doigts et à la paume de la main. Les organes de commande supportés par la poignée sont les suivants : sur la face antérieure, une détente 14 actionnable par l'index commande la mise sous tension soit d'un coagulateur thermique ou électrique, soit d'un générateur Laser, soit de tout autre instrument associé à l'appareil, sur la face postérieure, un couple de poussoirs 15 et 16 commandent respectivement l'aspiration et l'injection de liquide d'irrigation ; il est important de noter que ces deux poussoirs étant voisins, ils peuvent être actionnés simultanément aussi bien que séparément, par le pouce ; sur chacune des faces latérales, une lumière 17,17′ permet le passage d'une manette 18 pour la commande manuelle de la mise en oeuvre d'instruments soit rigides soit souples, ces manettes pouvant être installées aussi bien pour la main droite que pour la main gauche. On remarquera aussi un canal 10 de passage de câbles de connexion électrique.

La seconde partie, inférieure, de l'appareil est constituée essentiellement par la canule proprement dite 20 et par une pièce de raccordement 21 solidaire par construction de la canule (suivant une autre forme de réalisation, la canule proprement dite pourrait être une pièce séparable de la pièce de raccordement) ; la pièce de raccordement 21 a la forme d'un prisme à bases polygonales 23,24, l'une des faces latérales constituant la face de raccordement 3′, une autre face latérale comprenant un évidement 25 pour l'encliquetage du cliquet 5, une autre face latérale 26 comportant deux ajutages 27 et 28 de passage de fluides.

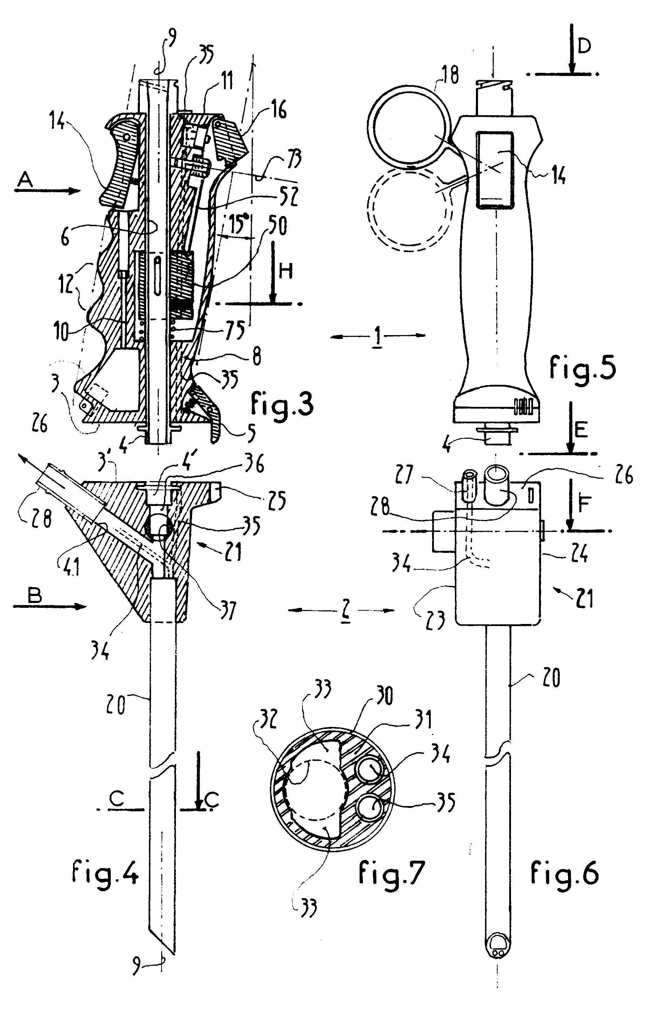

Sur la fig.7, il apparaît que la canule est composée d'un tube extérieur ou chemise 30 entourant trois conduits séparés par une matière plastique ou céramique 31 ; le conduit le plus important a une section de forme complexe comprenant une partie centrale circulaire 32 et deux parties latérales excentrées 33 : la partie centrale forme un conduit de guidage 32 pour des instruments rigides, eux-mêmes de section circulaire ; le deux parties latérales 33 constituent des conduits de retour d'irrigation ou d'aspiration ; un autre des conduits plus étroits est un conduit dit d'irrigation 34, tandis que le troisième est un conduit de passage 35 d'instruments souples.

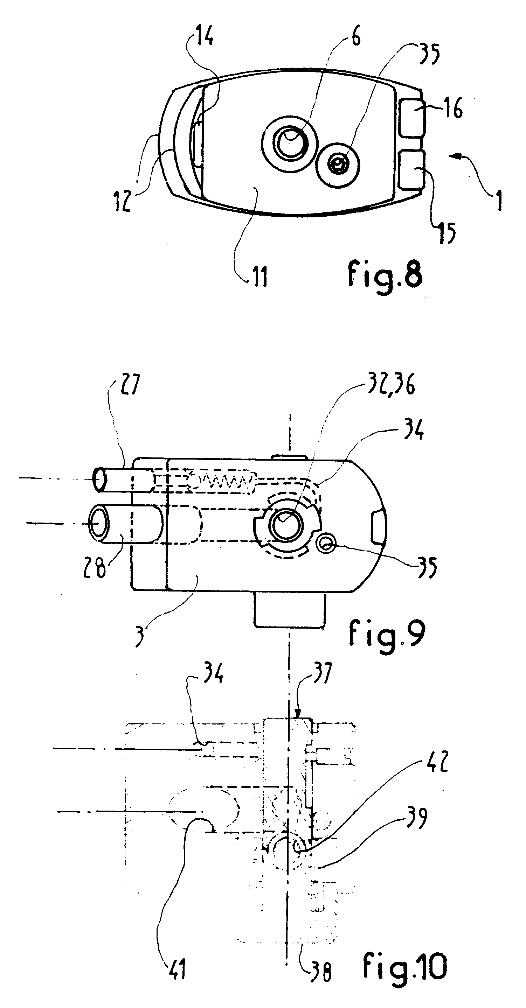

En se rapportant plus particulièrement aux fig.3,4,7,8,9 et 10, il apparaît comment le conduit de guidage 32 se sépare, dans la pièce de raccordement, du conduit d'évacuation 33, en un conduit latéral 41 qui débouche à l'extérieur de la pièce de raccordement 21. Une vanne à tiroir 37 pourvu d'un alésage 42 est disposée en travers du conduit axial 36 pour. Alternativement sous l'action d'un poussoir 38 à l'encontre d'un ressort 39, ouvrir ou fermer le conduit axial. Toujours dans la pièce de raccordement 21, le conduit d'irrigation 34 forme un coude (fig.9) pour rejoindre l'ajutage 27 ; cet ajutage 27 incorpore un clapet à bille 40 ne pouvant s'ouvrir à l'encontre d'un ressort qui si une pression suffisante du liquide d'irrigation est appliquée à l'ajutage.

La fig.9 montre comment débouche à la face de raccordement 3′ le conduit 35 d'instruments souples, qui débouche aussi (fig.8) à la face d'extrémité supérieure 11 de la poignée 1.

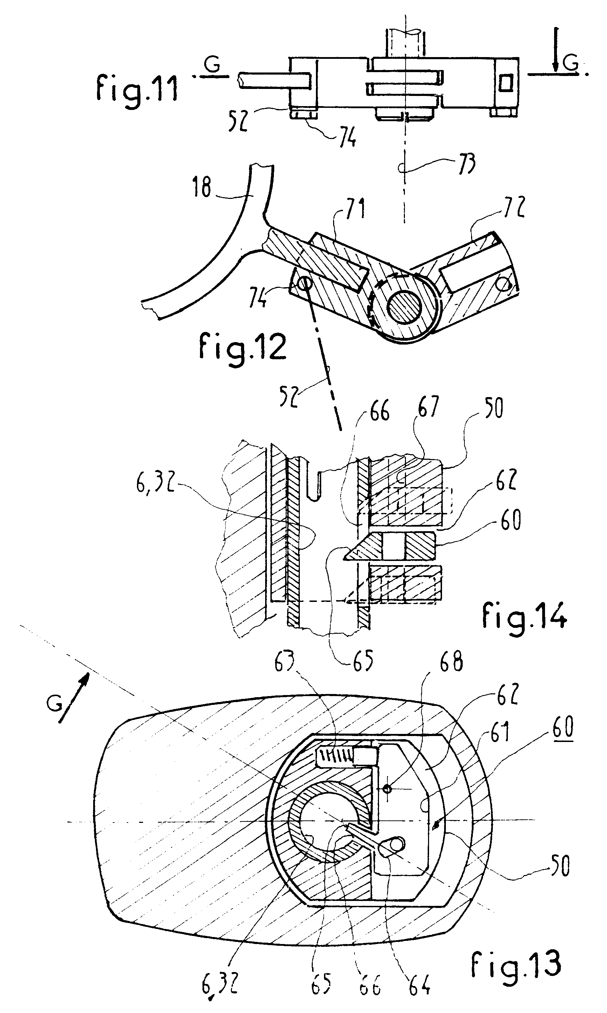

En relation avec les fig.11 à 18 plus particulièrement, va être expliquée la manoeuvre de divers instruments utilisables avec l'appareil. Les moyens de manoeuvre comprennent essentiellement un coulisseau 50 déplaçable axialement en va-et-vient par la manette 18 et une biellette 52 reliant le coulisseau à la manette (fig.3).

Le coulisseau peut faire prise sur des instruments de deux catégories : des instruments rigides pouvant être introduits dans le conduit de guidage 6,32, et des instruments souples pouvant être introduits dans le conduit de passage 35.

La prise du coulisseau sur les instruments souples est réalisée par le moyen d'un mécanisme d'accouplement 60 qui comprend un pièce 61, dite loquet, logée dans un évidement latéral 62 du coulisseau et montée pivotante autour d'un axe 68 à l'encontre d'un ressort 63 ; le loquet comporte un orifice oblongue 64 et un doigt palpeur 65 destiné à faie saillie à travers une lumière 66 dans l'espace intérieur du conduit de guidage 6,32 ; le coulisseau 50 est lui-même percé d'un conduit axial 67 situé dans le prolongement du conduit de passage 35 ; l'orifice 64 peut, par pivotement du loquet être placé en regard du conduit 66, lorsqu'un instrument rigide, présent dans le conduit de guidage 6,32, repousse le doigt palpeur 65 ; si un instrument souple est présent dans le conduit 35 et dans le conduit axial 67 du coulisseau, et qu'aucun instrument rigide n'est présent dans le conduit de guidage 6,32, alors le loquet fait pression sur l'instrument souple et couple celui-ci au coulisseau ; dès lors, la manoeuvre du coulisseau en va-et-vient entraînera le va-et-vient de l'instrument souple. On remarquera aussi à l'examen de la fig.14 que la position haute du coulisseau, en raison de la forme en plan incliné du doigt palpeur 65, libère nécessairement l'instrument souple. Le coulisseau 50 est en permanence sollicité en position haute par un ressort. Il résulte de cet ensemble de dispositions qu'un instrument souple et un instrument rigide non compatible ne peuvent jamais faire saillie ensemble involontairement à l'extrémité inférieure de la canule.

Revenant aux fig.11 et 12, on remarque que la manette 18 est montée en prolongement de l'un de deux bras de levier 71,72 articulés autour d'un axe 73, l'arbre sur lequel est monté la manette étant lui-même articulé à la biellette 52 autour d'un axe 74.

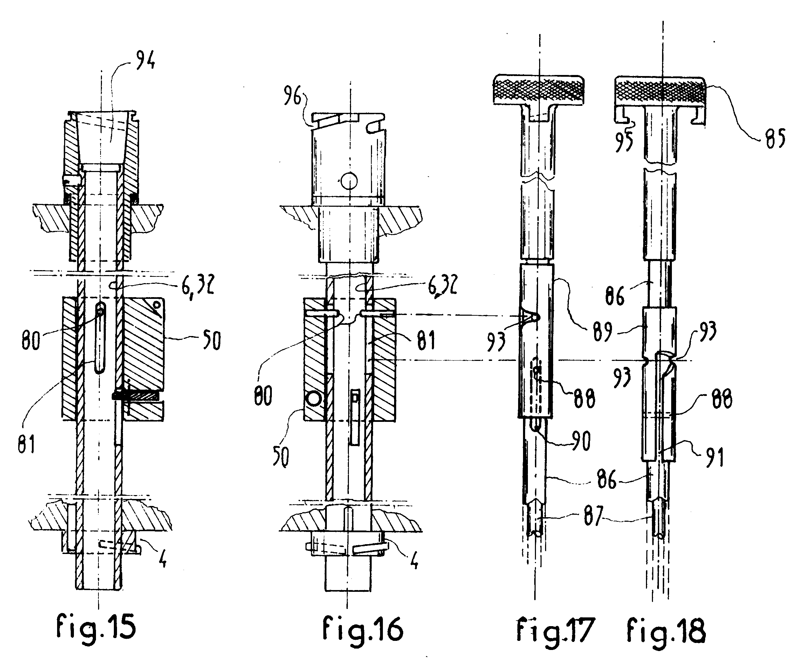

La prise du coulisseau sur les instruments dits rigides tel que cela ressort bien des fig.15 à 18 est réalisée par le moyen d'un couple d'ergots intérieurs 80 faisant une légère saillie à l'intérieur du conduit de guidage 6,32, à travers un couple de lumières 81 (fig.15,16). Les fig.17,18 plus particulièrement illustrent la partie supérieure d'un instrument rigide comprenant une tête 85 solidaire d'un tube 86 se prolongeant jusqu'au voisinage de l'extrémité de l'instrument (non représenté sur la figure) ; une tige 87 peut coulisser à l'intérieur du tube 86 et se prolonge jusqu'au voisinage de l'extrémité de l'instrument ; le tube 86 et la tige 87 sont reliés respectivement à une mâchoire de la partie fonctionnelle de l'instrument, par exemple machoire de pinces coupantes ou de pinces préhensiles. La tige 87 est reliée par une goupille 88 à un organe tubulaire 89 entourant le tube 86 en passant à travers un couple de lumières telles que 90 de ce dernier ; ainsi le mouvement de l'organe tubulaire 89 le long du tube 86 induit un mouvement correspondant de la tige 87 à l'intérieur de ce dernier ; on notera que les mouvements des pièces sus-décrites ne peuvent être que des mouvements axiaux, la rotation étant interdite par la goupille et les lumières 90 ; l'organe tubulaire est pourvu d'un couple de rainures transversales telles que 91 diamétralement opposées débouchant chacune dans une rainure transversale 93, l'ensemble de chaque rainure longitudinale 91 et transversale 93 formant une rainure en L renversé. L'introduction d'un instrument rigide par l'orifice supérieur 94 de l'appareil s'effectue en faisant glisser les rainures 91 de l'organe tubulaire le long des ergots 80 puis en faisant effectuer une rotation de 90° à la tête de l'instrument ; au cours de ce mouvement de rotation des griffes 95 de la tête de l'instrument viennent faire prise dans les rampes 96 dans un embout de l'orifice 94, tel qu'un embout "baïonnette-quart de tour" de type connu. Dans cette dernière position, les ergots 80 en prise dans le fond des rainures transversales 93 de l'organe tubulaire 89 entraîneront cet organe dans un déplacement correspondant à celui du coulisseau 50 sous la commande de la manette 18 non représentée sur les figures. On doit remarquer en se reportant aussi à la fig.10 que la présence du tube 86 dans l'alésage 42 du tiroir de la vanne assure la séparation étanche entre les parties inférieure et supérieure de l'appareil

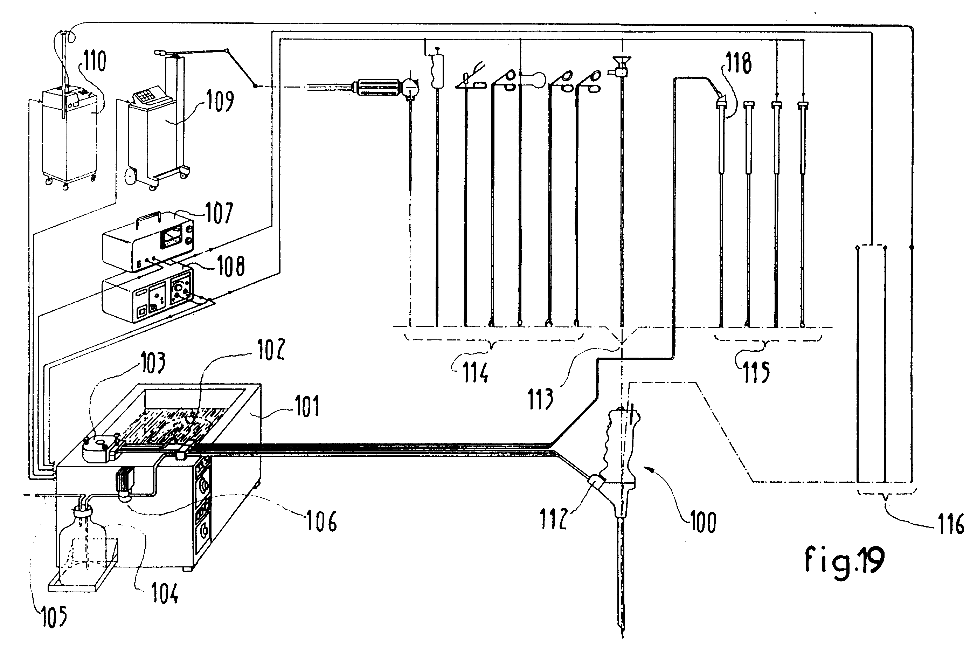

L'installation représentée à la fig.19 permettant une mise en oeuvre optimale de l'appareil 100 de l'invention regroupe une unité de conditionnement du fluide d'irrigation, une unité d'aspiration et de réception des liquides et tissus ponctionnés ainsi que l'ensemble des commandes principales d'irrigation, d'aspiration,d'électrocoagulation et de génération des rayons Laser.

L'unité de conditionnement du fluide d'irrigation comprend essentiellement un bain-marie 101 contenant une ou plusieurs poches souples telles que 102 de contention stérile du fluide d'irrigation dans lesquelles peuvent puiser des pompes péristaltiques telles que 103 qui refoulent dans des ajutages tels que 27 sus-décrit, ou encore dans d'autres instruments tels que l'instrument 118 ; accessoirement supporté par le bain-marie un bocal 104 de réception est en relation avec une prise de vide 105 et une ou plusieurs électrovannes telles que 106 reliées à des ajutages tels que 28 sus-décrit.

L'installation comprend en outre des alimentations 107 et 108 pour les instruments d'électrocoagulation ou de thermocoagulation, ainsi que des générateurs 109 et 110 de faisceaux Laser de type CO₂ ou Yag.

On remarquera la présence sur l'appareil 100 d'un bloc de raccordement 112 effectuant le raccordement de l'appareil non seulement pour les arrivées et départs de fluides mais aussi pour le commandes électriques émanant de divers organes de commande que supporte la poignée ; il est indiqué que toutes le commandes électriques sont en basse tension continue (DC 24 V).

On a enfin regroupé sur la fig.19 des instruments utilisables par l'appareil en deux catégories : d'une part, un ensemble d'instruments rigides 113 subdivisé en instruments conventionnels 114 et en instruments dits spécifiques 115, ces derniers étant du type représenté aux fig.17,18 et d'autre part, d'instruments souples 116, les instruments souples et les instruments rigides conventionnels étant connus de l'art de la coelioscopie opératoire.

## Revendications

1. Appareil chirurgical destiné à la coelioscopie opératoire, dit instrument multifonctionnel, constitué d'un tube, à l'intérieur duquel peuvent être introduits différents instruments, ledit tube pouvant permettre le passage de liquide corporel ou d'irrigation et comportant au voisinage de son extrémité, dite externe, un ensemble, dit poignée (8), de manoeuvre et de raccordement;
- ledit tube est constitué d'au moins deux parties :
- une première partie (6) entourée par ladite poignée (8),
- une deuxième partie formant une canule (20),
les deux parties pouvant être raccordées l'une à l'autre, dans leur prolongement axial, ou alternativement, être séparées l'une de l'autre ;
- ladite poignée (8) supporte une pluralité de moyens de commandes des diverses fonctions, actionnables simultanément ou séparément par deux doigts de la même main, à savoir :
. des premiers (16) et deuxièmes (15) moyens de commande électrique de l'envoi de liquide d'irrigation d'une part, et d'aspiration du liquide d'irrigation ou de liquides corporels d'autre part,
. des troisièmes moyens (14) de commande électrique, soit d'un coagulateur thermique ou électrique, soit d'un laser chirurgical,
. des moyens (18), dits quatrièmes, de commande mécanique d'instruments rigides dits spécifiques, et/ou d'instruments dits souples,
d'où il résulte que l'appareil peut recevoir simultanément des instruments dits souples (pointe électrocoagulante monopolaire, lame coupante, fibre optique conductrice de faisceau Laser, etc...) et des instruments rigides qui peuvent être soit des instruments conventionnels (pinces, ciseaux, Laser CO₂, ponction étanche, etc...) comportant leurs propres moyens de manoeuvre, soit des instruments dits spécifiques, conçus pour être manoeuvrés depuis la poignée ;

2. Appareil selon la revendcation 1, caractérisé :
en ce que la canule comporte au moins trois conduits le long de son axe :
- un conduit (34) dit d'irrigation,
- un conduit (35), dit de passage d'instruments de sectionnement souples de petit diamètre,
- un conduit (32) dit de guidage d'instruments rigides, formant aussi conduit d'évacuation (33), et
en ce que la canule (7) est solidaire d'une pièce dite de raccordement (21), approximativement en forme générale de prisme à base polygonale et comportant plusieurs faces latérales et deux faces de base, l'une (3') des faces latérales, perpendiculaire à l'axe (9) de la canule constituant une face de raccordement à la poignée ;

3. Appareil selon la revendication 2, caractérisé :
en ce que le conduit de guidage bifurque dans la pièce de raccordement :
- d'une part en un conduit axial (36) prolongeant le conduit de guidage (32) et émergeant à la face de raccordement,
- d'autre part, en un conduit (41), dit latéral, d'évacuation émergeant en un premier ajutage (28) sur une première face latérale (26), dite de branchement, de la pièce de raccordement,
en ce qu'une vanne à tiroir (37) pouvant alternativement obturer ou libérer le passage dans le conduit axial, et disposé en travers du conduit axial (36), l'axe du tiroir de la vanne étant perpendiculaire à l'axe du conduit axial et suffisamment proche de la bifurcation précédente pour ne pas créer de volume mort de rétention,
tandis qu'un clapet (40), situé dans un second ajutage (27), dit d'entrée d'irrigation, rend unidirectionnel vers la canule le passage de liquide dans le conduit d'irrigation (34), tout en garantissant une pression minimale d'irrigation lequel jet d'irrigation peut servir à détacher des adhérences fraiches ;

4. Appareil selon la revendication 3, caractérisé :
en ce que le conduit de guidage comporte une partie cylindrique (32) réservée au passage d'instruments, et au moins un couloir latéral (33) ménagé pour l'évacuation des liquides ;

5. Appareil selon la revendication 3, caractérisé :
en ce que ledit conduit de passage (35) d'instruments souples débouche à ladite face de raccordement (3') en coïncidence, lorsque les deux parties de l'appareil sont raccordées, avec son prolongement dans la première partie de la poignée jusqu'à son débouché à une extrémité supérieure de cette dernière ;

6. Appareil selon l'une quelconque des revendications précédentes, caractérisé :
en ce que la poignée (1) a la forme générale d'un parallélépipède allongé dont l'une des faces de base constitue une face de raccordement (31), et est perpendiculaire à ladite première partie du tube,
en ce que l'axe de la poignée est incliné d'environ 15° par rapport à l'axe du tube principal,
en ce qu'au moins un bouton poussoir (15,16) est disposé sur une face, dite postérieure, de la poignée tandis qu'un autre poussoir, dit détente (14), est disposé sur une face dite antérieure de la poignée, poussoirs et détente étant situés au voisinage de l'extrémité supérieure de cette dernière, et
en ce qu'une manette (18) de manoeuvre est disposée sur l'une des faces latérales de la poignée au voisinage de l'extrémité supérieure de celle-ci ;

7. Appareil selon la revendication 6, caractérisé :
en ce que ladite manette est reliée à un coulisseau (50) entourant ladite première partie (6) du tube dans un logement approprié de la poignée en vue de communiquer par l'intermédiaire du coulisseau et d'au moins un ergot intérieur (80) de celui-ci passant à travers une lumière longitudinale (81) pratiquée dans le tube principal, un mouvement axial à un organe d'un instrument placé dans le tube principal ;

8. Appareil selon la revendication 7, caractérisé :
en ce que le coulisseau (50), percé lui-même axialement (67) dans le prolongement dudit conduit de passage (35) d'instruments souples, supporte un mécanisme (60,63,64,65) d'accouplement destiné à accoupler au coulisseau un instrument souple passant dans le conduit de passage et permettre la manoeuvre en va-et-vient par l'intermédiaire du coulisseau et de la manette dudit instrument souple, ou au contraire à découpler l'instrument souple du coulisseau en cas de présence dans le tube principal d'instruments rigides non compatibles avec l'instrument souple, le couplage ou le découplage étant opéré par un doigt palpeur (65) faisant saillie à travers une lumière (66) dans le tube principal ;

9. Appareil selon l'une quelconque des revendications 3 à 8, caractérisé :
en ce que le raccordement de la poignée à la canule est réalisé par le moyen d'un emboîtement à baïonnette (4) "quart de tour" et rampe hélicoïdale (4'), un mécanisme de verrouillage (5,25) par cliquet (5) assurant le maintien en position raccordée des deux parties (1,2) de l'appareil ;

## Claims

1. Surgical apparatus intended for operative coelioscopy, a so-called multipurpose instrument, comprising a tube within which different instruments may be introduced, the said tube allowing the passage of body fluid or irrigation fluid and comprising in the vicinity of its extremity, referred to as the outer end, an operating and connecting assembly called a handle (8):
- the said tube comprises at least two parts:
- a first part (6) surrounded by the said handle (8),
- a second part forming a cannula (20),
- it being possible for the two parts to be attached to each other along their axial extent, or alternatively being separate from one another,
- the said handle (8) supports a plurality of controls for various functions which can be operated simultaneously or separately by two fingers of the same hand, namely:
· first (16) and second (15) means for electrical control of the delivery of irrigation fluid on the one hand, and aspiration of the irrigation fluid or body fluids on the other,
· third means (14) for electrical control of a thermal or electrical coagulator, or a surgical laser,
· means (18), referred to as fourth means, for mechanically controlling rigid instruments referred to as specific instruments, and/or instruments which are referred to as flexible,
as a result whereof the apparatus can simultaneously receive instruments which are referred to as flexible (monopolar electrocoagulating tip, cutting blade, optical fibre conducting a laser beam, etc.) and rigid instruments which may be either conventional instruments (forceps, scissors, CO₂ laser, sealed puncture device, etc.) which have their own means of control, or instruments referred to as specific instruments, which are designed to be operated from the handle.

2. Apparatus according to Claim 1, **characterised in that** the cannula possesses at least three ducts along its axis:
- a duct (34), referred to as an irrigation duct,
- a duct (35), called a duct for the passage of the flexible small-diameter cutting instruments,
- a duct (32) called a guiding duct for rigid instruments, also forming an evacuation duct (33), and wherein the cannula (7) is integral with a piece referred to as a connecting piece (21), having approximately the general shape of a polygonal based prism and possessing a plurality of lateral faces and two base faces, one (3') of the lateral faces, perpendicular to the axis (9) of the cannula, constituting a face for connection to the handle.

3. Apparatus according to Claim 2, **characterised in that** the guiding duct forks in the connecting piece:
- on the one hand into an axial duct (36) extending the guiding duct (32) and emerging at the connecting face,
- on the other hand into an evacuation duct (41), referred to as lateral, emerging in a first nozzle (28) on a first lateral face (26) referred to as a branching face on the connecting piece,
wherein a slide valve (37) which can alternatively close or open the passage in the axial duct, and is arranged across the axial duct (36), the axis of the slide of the valve being perpendicular to the axis of the axial duct and sufficiently close to the above mentioned fork not to create a dead retention volume,
while a valve (40), situated in a second nozzle (27), referred to as an irrigation inlet, renders the passage of liquid in the irrigation duct (34) monodirectional, while guaranteeing a minimum irrigation pressure which irrigation jet can serve to detach fresh adhesions.

4. Apparatus according to Claim 3 **characterised in that** the guiding duct possesses a cylindrical part (32) reserved for the passage of instruments, and at least one lateral passage (33) provided for the evacuation of the liquids.

5. Apparatus according to Claim 3, wherein the said duct (35) for the passage of flexible instruments terminates at the said connection face (3'), coinciding, when the two parts of the apparatus are connected, with its extension into the first part of the handle as far as its termination at an upper end of the latter.

6. Apparatus according to any one of the preceding Claims, wherein the handle (1) has the general shape of an elongate parallelepiped one of whose base faces forms a connecting face (31), and is perpendicular to the said first part of the tube, wherein the axis of the handle is inclined at about 15° relative to the axis of the main tube, wherein at least one push-button (15,16) is provided on a face, referred to as a posterior face of the handle while another push-button, called the detent (14), is provided on a face referred to as the anterior face of the handle, the push-buttons and detent being situated in the vicinity of the upper end of the latter, and wherein an operating lever (18) is provided on one of the lateral faces of the handle in the vicinity of the upper end of the latter.

7. Apparatus according to Claim 6, wherein the said lever is connected to a slider (50) surrounding the said first part (6) of the tube in an appropriate seating in the handle with a view to imparting, via the slider and at least one internal pin (80) of the latter passing through a longitudinal port (81) made in the main tube, an axial movement to a member of an instrument placed in the main tube.

8. Apparatus according to Claim 7, **characterised in that** the slider (50), itself pierced axially (67) in the extension of the said duct (35) for the passage of flexible instruments, supports a coupling mechanism (60, 63, 64, 65) intended to couple to the slider a flexible instrument passing into the passage duct and to permit the reciprocal manipulation of the said flexible instrument via the slider and the lever, or on the other hand to uncouple the flexible instrument from the slider in the event of the presence in the main tube of rigid instruments which are incompatible with the flexible instrument, the coupling or uncoupling being performed by a sensor finger (65) which projects through a port (66) in the main tube.

9. Apparatus according to any one of Claims 3 to 8, **characterised in that** the connection of the handle to the cannula is achieved by means of a "quarter-turn" bayonet connector (4) with helical ramp (4'), a ratchet (5) locking mechanism (5, 25) ensuring that the two parts (1,2) of the apparatus are maintained in the connected position.

## Patentansprüche

1. Vorrichtung zur operativen Coelioskopie als sogenanntes multi-funktionales Instrument, gebildet aus einer Hülse, in die verschiedene Instrumente eingeführt werden können, und die das Hindurchtreten von Körperflüssigkeit oder Spülflüssigkeit erlaubt, und im Bereich seines Endes, des sogenannten äußeren Endes, eine Griffeinheit (8) zum Manövrieren und Anschließen;
- wobei die genannte Hülse aus wenigstens zwei Abschnitten besteht;
- einem ersten Abschnitt (6), der vom Handgriff (8) umgeben ist;
- einem zweiten Abschnitt, der eine Kanüle (20) bildet;
- wobei die beiden Abschnitte in ihrer Axialerstreckung miteinander verbunden oder alternativ voneinander getrennt werden können;
- wobei der Handgriff (8) eine Mehrzahl von Steuermitteln für verschiedene Funktionen trägt, die gleichzeitig oder getrennt durch zwei Finger derselben Hand betätigt werden können, nämlich:
* erste (16) und zweite (15) elektrische Steuermittel zum Zuführen von Spülflüssigkeit einerseits und zum Abziehen von Spülflüssigkeit oder Körperflüssigkeit andererseits;
* dritte elektrische Steuermittel (14) seien diese für einen thermischen oder elektrischen Coagulator oder für einen chirurgischen Laser;
* sogenannte vierte Steuermittel (18) für mechanische, sogenannte steife, sogenannte spezifische Instrumente und/oder sogenannte weiche Instrumente,
so daß die Vorrichtung gleichzeitig sogenannte weiche Instrumente aufzunehmen vermag (monopolare elektrocoagulierende Spitze, Schneidklinge, optisch leitende Fasern des Laserbündels, usw.) sowie starre Instrumente, die entweder herkömmliche Instrumente sein können (Pinzetten, Scheren, Laser CO₂, dichte Einstiche, usw...), die ihre eigenen Manövriermittel aufweisen, oder sogenannte spezifische Instrumente, die mittels des Handgriffes manövriert werden können.

2. Vorrichtung gemäß Anspruch 1, dadurch gekennzeichnet, daß die Kanüle wenigstens drei Leitungen entlang ihrer Achse aufweist:
- eine sogenannte Spülleitung (34), eine Leitung (35) zum Hindurchführen von weichen Instrumenten kleinen Durchmessers;
- eine Führungsleitung (32), die auch eine Evakuierungsleitung (33) bildet, für starre Instrumente, und
- daß die Kanüle (7) einteilig mit einem Verbindungsstück (21) ist, das annähernd eine prismatische Form auf polygonaler Basis hat und mehrere Seitenflächen sowie zwei Basisflächen aufweist, wobei eine (3') der Seitenflächen, die senkrecht zur Achse (9) der Kanüle verläuft, eine Verbindungsfläche zum Handgriff bildet.

3. Vorrichtung gemäß Anspruch 2, dadurch gekennzeichnet, daß sich die Führungsleitung im Anschlußstück verzweigt:
- einerseits in eine Axialleitung (36), die die Führungsleitung (32) verlängert und an der Verbindungsfläche austritt,
- andererseits in eine seitliche Leitung (41) zum Evakuieren, die in einem ersten Stutzen (28) auf einer ersten Seitenfläche, der sogenannten Verzweigungsfläche des Anschlußstückes austritt, wobei ein Schieberventil (37) teilweise die Axialleitung absperren oder freigeben kann und in der Axialleitung angeordnet ist, wobei die Achse des Schieberventils senkrecht zur Achse der Axialleitung verläuft und genügend nahe bei der Verzweigung angeordnet ist, um keine toten Ecken entstehen zu lassen,
wobei ein Klappenventil (40), das in einer zweiten Hülse (27), dem sogenannten Spüleingang, angeordnet ist, den Flüssigkeitsdurchgang in der Spülleitung (34) gegen die Kanüle hin in einer Richtung gestaltet und dabei einen minimalen Spüldruck gewährleistet, wobei der Spülstrahl dazu dient, neue Anhaftungen zu lösen.

4. Vorrichtung gemäß Anspruch 3, dadurch gekennzeichnet, daß die Führungsleitung einen zylindrischen Teil (32) für die Hindurchführung von Instrumenten aufweist, sowie wenigstens einen Seitenkanal (33) zur Evakuierung von Flüssigkeiten.

5. Vorrichtung gemäß Anspruch 3, dadurch gekennzeichnet, daß die Leitung (35) für weiche Instrumente in der Anschlußfläche (3') und damit zusammenfällt, während die beiden Teile der Vorrichtung aneinander angeschlossen sind, mit der Verlängerung im ersten Teil des Handgriffs bis zur Mündung an einem oberen Ende des letztgenannten.

6. Vorrichtung gemäß einem der vorausgegangenen Ansprüche, dadurch gekennzeichnet, daß der Handgriff (1) im wesentlichen die Form eines langgestreckten Parallel-Epipeds aufweist, dessen eine Basisfläche eine Anschlußfläche (31) bildet, und senkrecht zum genannten ersten Teil der Hülse verläuft,
daß die Achse des Handgriffs unter etwa 15° in die Achse der Haupthülse geneigt ist,
und daß wenigstens ein Druckknopf (15, 16) an einer Fläche, der sogenannten hinteren Fläche, des Handgriffs vorgesehen ist, während ein weiterer Druckknopf, der sogenannte Freigabeknopf (14) an einer Fläche, der sogenannten vorderen Fläche des Handgriffs vorgesehen ist, wobei Druckknopf und Freigabeknopf im Bereich des oberen Endes des Handgriffs angeordnet sind,
und daß ein Bedienungshebel (18) an einer der Seitenflächen des Handgriffs im Bereich von dessen oberem Ende vorgesehen ist.

7. Vorrichtung gemäß Anspruch 6, dadurch gekennzeichnet, daß der Bedienungshebel an eine Kulisse (50) angeschlossen ist, die das genannte erste Teil (6) der Hülse in einer Ausnehmung des Handgriffes umgibt, um mittels der Kulisse und wenigstens eines Innenstiftes der Kulisse, hindurchgeführt durch ein Langloch (81) in der Haupthülse, eine Axialbewegung auf ein Organ eines Instrumentes zu übertragen, das in der Haupthülse angeordnet ist.

8. Vorrichtung gemäß Anspruch 7, dadurch gekennzeichnet, daß die Kulisse (50), die ihrerseits axial (67) in der Verlängerung der Führungsleitung (35) für weiche Instrumente durchbohrt ist, einen Anschlußmechanismus (60, 63, 64, 65) zum Ankoppeln eines durch die Leitung hindurchgeführten Instrumentes an die Kulisse vorzunehmen und um das Hin- und Hergehen des weichen Instrumentes mittels der Kulisse und des Handhebels zu ermöglichen, oder um statt dessen das weiche Instrument von der Kulisse abzukoppeln, falls sich in der Haupthülse starre Instrumente befinden, die sich mit den weichen Instrumenten nicht vertragen, wobei das Ankoppeln und Abkoppeln mittels eines Tasters (65) vorgenommen wird, der durch eine Aussparung (66) in der Haupthülse hindurchgeführt ist.

9. Vorrichtung gemäß einem der Ansprüche 3-8, dadurch gekennzeichnet, daß das Anschließen des Handgriffes die Kanüle mittels eines Bajonettverschlusses (4) mit einer Viertelumdrehung und mittels einer schraubenlinienförmigen Rampe (4') vorgenommen wird, wobei ein Verriegelungsmechanismus (5, 25) mit einer Klinke (5) das Festhalten der beiden Teile (1, 2) der Vorrichtung in angeschlossenem Zustand sicherstellt.
